# EUROPÄISCHE PATENTSCHRIFT

(11) **EP 3 001 816 B1**
(45) Veröffentlichungstag und Bekanntmachung des Hinweises auf die Patenterteilung: **20.12.2017**
(21) Anmeldenummer: 14723393.6
(22) Anmeldetag: 30.04.2014
(51) Int. Cl.: A61L 2/22, A61L 2/24, A61L 2/07, A47L 15/00, A47L 15/42, A61G 9/02, F04D 1/06

(54) **VORRICHTUNG ZUR REINIGUNG VON BEHÄLTERN**
DEVICE FOR CLEANING CONTAINERS
DISPOSITIF DE NETTOYAGE DE CONTENANTS

(30) Priorität: 02.05.2013 DE 102013208060
(43) Veröffentlichungstag der Anmeldung: 06.04.2016
(73) Patentinhaber: MEIKO Maschinenbau GmbH & Co. KG, 77652 Offenburg (DE)
(72) Erfinder: GAUS, Bruno, 77654 Offenburg (DE)
(74) Vertreter: Herzog, Fiesser & Partner Patentanwälte PartG mbB
(86) Internationale Anmeldenummer: PCT/EP2014/058895
(87) Internationale Veröffentlichungsnummer: WO 2014/177648

(56) Entgegenhaltungen:
- EP-A1- 0 516 563
- EP-A2- 0 341 766
- WO-A1-2005/072595
- WO-A2-2012/107332
- DE-A1- 3 308 230
- DE-A1- 10 348 344

## Beschreibung

### Gebiet der Erfindung

Die Erfindung betrifft eine Reinigungsvorrichtung sowie ein Verfahren zur Reinigung mindestens eines Behälters zur Aufnahme von menschlichen Ausscheidungen. Derartige Reinigungsvorrichtungen und Verfahren werden beispielsweise im Krankenhaus- oder Pflegebereich eingesetzt, um Behälter wie beispielsweise Nachtgeschirr, Steckbecken, Bettpfannen oder andere Behälter zu reinigen und optional zu desinfizieren, welche zur Aufnahme menschlicher oder tierischer Ausscheidungen geeignet sind. Beispielsweise werden derartige Reinigungsvorrichtungen als Reinigungs- und Desinfektionsgeräte eingesetzt.

### Stand der Technik

Aus dem Stand der Technik sind eine Vielzahl von Reinigungsvorrichtungen und Reinigungsverfahren zur Behandlung von Behältern zur Aufnahme menschlicher Ausscheidungen bekannt. Die zu behandelnden Behälter können größere Flüssigkeitsmengen oder Mengen an Feststoffabfällen enthalten, welche üblicherweise während der Reinigung entsorgt werden müssen. Zudem können derartige Behälter infektiöse Abfälle enthalten oder anderweitig kontaminiert sein, so dass, neben einer Entleerung, auch üblicherweise eine Desinfektion erforderlich ist. Derartige Reinigungsgeräte zur Behandlung von Behältern für menschliche Ausscheidungen werden dementsprechend häufig auch als Reinigungs- und Desinfektionsgeräte (RDG) bezeichnet.

Weiter können bei solchen Behältern starke Verschmutzungen auftreten, so dass während der Reinigung, zusätzlich zu einer üblichen Entleerung, eine erhöhte Abtragsleistung erforderlich ist. Insbesondere kann es erforderlich sein, die Behälter zusätzlich zu einer Beaufschlagung mit einem Reinigungsfluid mit Niederdruck durch eine Behandlung mit stärkerer Abtragsleistung, insbesondere durch eine Beaufschlagung mit einem Reinigungsfluid mit Hochdruck, zu reinigen.

Neben den genannten Behältern sind diese Reinigungsvorrichtungen grundsätzlich auch zur Reinigung anderer medizinischer Gegenstände geeignet, wie sie beispielsweise in Krankenhäusern oder in Altenpflegeeinrichtungen eingesetzt werden. Üblicherweise besteht das Reinigungsgut jedoch aus Urinflaschen, Steckbecken, Nierenschalen, Waschschüsseln oder ähnlichen Behältern, deren Reinigung die Entsorgung größerer Abfallmengen mit sich bringen kann.

Beispielsweise kann eine Reinigungsvorrichtung wie in DE 10 348 344 B4 beschrieben ausgestaltet sein. In DE 103 48 344 B4 wird eine Vorrichtung zur Durchführung eines Verfahrens zum Abkühlen von desinfiziertem Reinigungsgut offenbart. Die Vorrichtung weist eine Kammer auf, die von einer Wasser-/Dampf-Einheit sowohl mit Kaltwasser oder Warmwasser als auch mit Wasserdampf beaufschlagbar ist. Der Kaltwasser- oder Warmwassereintrag erfolgt über Sprühdüsen. In der Vorrichtung läuft ein Wasch- oder Reinigungsprogramm ab, welches Reinigungsschritte und einen Desinfektionsschritt umfasst.

Weiter sind aus dem Stand der Technik, beispielsweise aus DE 10 348 344 B4, Reinigungsvorrichtungen bekannt, welche eine Pumpe aufweisen, die eingerichtet ist, das Reinigungsfluid unter Druck zu mindestens einer Düse in einer Reinigungskammer zu fördern, durch welche ein in der Reinigungskammer befindliches Reinigungsgut mit Reinigungsfluid beaufschlagt werden kann. Die Pumpe fördert das Reinigungsfluid beispielsweise aus einem Sammelraum am Boden der Reinigungskammer oder aus einem anderen Vorratsbehälter zu der mindestens einen Düse.

DE 198 12 231 A1 offenbart eine Geschirrspülmaschine mit einer Spülflüssigkeitspumpe und zwei Sprüharmen, wobei mittels Ventilen der Druck der Sprüharme unterschiedlich eingestellt werden kann. DE 195 21 21 536 C2 beschreibt einen Hochdruckreiniger mit zwei wahlweise betätigbaren Pumpen. DE 33 08 230 A1 beschreibt ebenfalls HochdruckReinigungsgeräte mit einem Hochdruckbetrieb und einem Niederdruckbetrieb. DE 1 503 853 beschreibt eine Geschirrspülmaschine mit einer mehrstufigen Kreiselpumpe, welche für zwei verschiedene Drehzahlen ausgebildet ist.

DE 103 48 344 A1 beschreibt ein Verfahren und eine Vorrichtung zur Rückkühlung von Reinigungsgut in Reinigungs- und Desinfektionsautomaten für menschliche Ausscheidungen aufnehmende Behälter. Innerhalb einer Kammer erfolgt ein Abspülen des in dieser enthaltenen Reinigungsgutes, woran sich ein Vorreinigungsspülschritt anschließt. Danach wird das in der Kammer enthaltene Reinigungsgut mit einem einen Klarspülzusatz enthaltenden Wasser endgereinigt, bevor ein Desinfektionsschritt des Reinigungsgutes in der Kammer durch Einleitung von Wasserdampf in diese erfolgt. In die mit Wasserdampf befüllte Kammer wird zwangsweise Luft bei geschlossener Tür eingebracht, wodurch ein Niederschlag von Wasserdampf innerhalb der Kammer sowie eine Abkühlung und eine Trocknung des in der Kammer enthaltenen Reinigungsgutes bewirkt wird.

EP 0516563 A1 beschreibt ein Wasch- und Desinfektionsverfahren für Schuhe. Bei diesem werden die Schuhe in eine Position gebracht, in welcher das Wasser aus diesen herausfließen kann. Anschließend wird ein Vorwaschgang durchgeführt, bei welchem die Schuhe unter Hochdruck mit Wasser, optional unter Zusatz eines Reinigers, besprüht werden. Anschließend wird ein Waschschritt durchgeführt, in welchem unter Niederdruck Wasser mit einem Reinigerzusatz sowie optional einem Desinfektionsmittelzusatz auf die Schuhe aufgesprüht wird. Anschließend wird ein Abspülschritt durchgeführt, in welchem unter Niederdruck Wasser auf die Schuhe aufgesprüht wird.

WO 2005/072595 A1 beschreibt einen Geschirrspüler mit zeitlich aufeinanderfolgend betriebenen oberen und unteren Sprüharmen. Weiterhin wird ein Steuerungsverfahren für den Geschirrspüler beschrieben, durch welches die Filtereffizienz verbessert wird, um die Waschleistung zu verbessern und um den Lärmpegel zu verringern. Dazu wird eine Variabilität der Anzahl der Umdrehungen der Zirkulationspumpe ausgenutzt.

Bei stark verschmutztem Reinigungsgut kann grundsätzlich eine Erhöhung des Drucks, mit dem das Reinigungsfluid aus einer Öffnung einer Beaufschlagungsvorrichtung, insbesondere einer Düse, austritt, erforderlich sein. Beispielsweise könnte diese Druckerhöhung durch ein in der Reinigungsvorrichtung vorgesehenes Hochdruck-Reinigungssystem realisiert werden. Eine Druckerhöhung bei Verwendung der gleichen Düse, wie in einer Reinigungsvorrichtung ohne Hochdrucksystem, könnte jedoch nachteilig sein, da sich ein Volumenstrom des verwendeten Reinigungsfluids ebenfalls erhöhen wird. Durch eine Verwendung einer Beaufschlagungsvorrichtung mit einer kleineren Öffnung kann der Volumenstrom grundsätzlich verringert werden. So kann mit Hochdruck-Reinigungssystemen bei einem kleineren Volumenstrom als beispielsweise bei Niederdruck-Reinigungssystemen ein gleicher oder sogar höherer Energieinhalt der versprühten Reinigungslösung und damit ein verbessertes Reinigungsergebnis erreicht werden. Beispielsweise könnten zur Verbesserung eines Reinigungsergebnisses mehrere Reinigungssysteme in der Reinigungsvorrichtung vorgesehen sein, beispielsweise ein Niederdruck- und ein Hochdruck-Reinigungssystem. Bei einem Einbau eines Niederdruck- und eines Hochdruck-Reinigungssystems in eine Reinigungsvorrichtung sind jedoch grundsätzlich ein erhöhter apparativer Aufwand, ein erhöhtes Bauvolumen und auch erhöhte Herstellungskosten durch erforderliche zusätzliche Bauteile zu erwarten. So müssten beispielsweise die Pumpe, ein Leitungssystem und die Beaufschlagungsvorrichtung, insbesondere Düsen, mehrfach eingebaut werden.

### Aufgabe der Erfindung

Es ist daher Aufgabe der vorliegenden Erfindung, eine Reinigungsvorrichtung und ein Verfahren zur Reinigung eines Behälters für menschliche Ausscheidungen bereitzustellen, welche die Nachteile bekannter Vorrichtungen und Verfahren zumindest weitgehend vermeiden. Insbesondere soll ein geringer apparativer Aufwand erreicht werden.

### Offenbarung der Erfindung

Diese Aufgabe wird gelöst durch eine Reinigungsvorrichtung und ein Verfahren zur Reinigung von Reinigungsgut mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Die beschriebene Reinigungsvorrichtung kann insbesondere eingerichtet sein, um ein erfindungsgemäßes Verfahren durchzuführen. Umgekehrt kann das Verfahren unter Verwendung der erfindungsgemäßen Reinigungsvorrichtung durchgeführt werden. Dementsprechend kann für die Beschreibung optionaler Details der Reinigungsvorrichtung auf die Beschreibung optionaler Details des Verfahrens verwiesen werden und umgekehrt.

In einem ersten Aspekt wird eine Reinigungsvorrichtung zum Reinigen von Reinigungsgut vorgeschlagen. Unter einer Reinigungsvorrichtung ist eine Vorrichtung zu verstehen, welche eingerichtet ist, um Verunreinigungen, die an dem Reinigungsgut anhaften und/oder darin enthalten sind, zumindest weitgehend zu beseitigen. Optional kann die Reinigungsvorrichtung zusätzlich noch eine keimabtötende Wirkung, beispielsweise eine Desinfektionswirkung, entfalten.

Die Reinigungsvorrichtung kann insbesondere ausgewählt sein aus der Gruppe bestehend aus einer Geschirrspülmaschine und einem Reinigungs- und Desinfektionsgerät zur Reinigung von Behältern zur Aufnahme menschlicher Ausscheidungen, insbesondere einem Steckbeckenspüler. Eine derartige Reinigungsvorrichtung zur Reinigung von Behältern zur Aufnahme menschlicher Ausscheidungen kann als Reinigungs- und Desinfektionsgerät bezeichnet werden. Die Reinigungsvorrichtung kann beispielsweise gemäß dem oben beschriebenen Stand der Technik ausgestaltet sein, mit den nachfolgend beschriebenen zusätzlichen erfindungsgemäßen Merkmalen. Auch eine andere Ausgestaltung ist jedoch möglich.

Unter Reinigungsgut können dabei allgemein im Rahmen der vorliegenden Erfindung ein oder mehrere Gegenstände verstanden werden, welche gereinigt werden sollen. Insbesondere kann das Reinigungsgut einen oder mehrere Behälter umfassen und weiter insbesondere einen oder mehrere Behälter zur Aufnahme menschlicher Ausscheidungen. Insbesondere kann die Reinigungsvorrichtung also zur Reinigung von Behältern zur Aufnahme menschlicher Ausscheidungen ausgestaltet sein. Beispielsweise kann der Behälter eingerichtet sein, um Ausscheidungen in einer Menge von mindestens 100 ml, insbesondere in einer Menge von mindestens 200 ml oder sogar mindestens 500 ml oder mehr, aufzunehmen. Das Reinigungsgut kann ausgewählt sein aus der Gruppe bestehend aus: Urinflaschen, Steckbecken, Nierenschalen, Waschschüsseln oder ähnlichen Behältern, welche beispielsweise in Krankenhäusern und Pflegeeinrichtungen eingesetzt werden.

Der Behälter kann mindestens einen Behälterrand aufweisen, welcher starr oder auch verformbar, insbesondere flexibel ausgestaltet sein kann. So kann der Behälter beispielsweise ein Gefäß mit einer starren Behälterwand, beispielsweise aus einem oder mehreren der Materialien Glas, Kunststoff, Keramik und Metall, aufweisen. Alternativ oder zusätzlich kann der Behälter auch mindestens eine verformbare Behälterwand, beispielsweise mindestens einen Folienbeutel aus einem Kunststoffmaterial, aufweisen. So kann der Behälter für menschliche Ausscheidungen beispielsweise auch ganz oder teilweise als zunächst geschlossenes Behältnis in Gestalt eines Folienbeutels ausgestaltet sein.

Der Behälter kann mindestens einen Aufnahmebereich für menschliche Ausscheidungen aufweisen, beispielsweise mindestens einen Hohlraum. Weiter kann der Behälter mindestens eine Öffnung aufweisen, welche von vorneherein vorhanden sein kann und/oder auch zu einem späteren Zeitpunkt geschaffen werden kann, beispielsweise während und/oder vor der Reinigung. Die Öffnung kann beispielsweise durch ein mechanisches Öffnen des Behälters und/oder durch ein Aufschneiden und/oder ein Aufreißen des Behälters geschaffen werden. Weiterhin kann die Öffnung eingerichtet sein, um reversibel oder irreversibel geöffnet und/oder verschlossen zu werden.

Die Reinigungsvorrichtung umfasst mindestens ein Düsensystem zur Beaufschlagung des Reinigungsguts mit mindestens einem Reinigungsfluid. Unter einem Düsensystem ist allgemein eine Vorrichtung zu verstehen, mit der mindestens ein Reinigungsfluid auf dem Behälter aufgebracht werden kann, vorzugsweise direkt, beispielsweise durch Besprühen, Betropfen oder Bestrahlen oder eine Kombination der genannten Beaufschlagungen und/oder auf eine andere Weise. Das Düsensystem kann eine oder mehrere Düsen aufweisen, beispielsweise eine Mehrzahl von Düsen, insbesondere mindestens zwei Düsen. Die Düsen können insbesondere jeweils mindestens eine Öffnung aufweisen, beispielsweise mindestens eine Sprühöffnung oder eine Mehrzahl von Sprühöffnungen, durch welche das Reinigungsfluid auf dem Behälter aufgebracht werden kann.

Unter einem Reinigungsfluid kann eine beliebige Flüssigkeit verstanden werden, welche eine reinigende Wirkung auf das Reinigungsgut aufweist. Beispielsweise kann das Reinigungsfluid Wasser oder eine wässrige Reinigungsflüssigkeit mit einem oder mehreren Zusatzstoffen, wie beispielsweise mindestens einem Reinigerkonzentrat und/oder mindestens einem chemischen Zusatz wie beispielsweise einem Desinfektionsmittel und/oder einem Klarspülmittel, sein. Allgemein können auch verschiedene Arten von Reinigungsfluiden eingesetzt werden.

Das Düsensystem weist mindestens eine Hochdruckdüse und mindestens eine Niederdruckdüse auf. Unter einer Düse ist dabei allgemeinem Rahmen der vorliegenden Erfindung eine Vorrichtung mit mindestens einer Öffnung, auch als Düsenöffnung oder Sprühöffnung bezeichnet, zu verstehen, welche eingerichtet ist, um einen oder mehrere Strahlen des Reinigungsfluids zu erzeugen. So kann es sich beispielsweise bei der Düse um eine Vorrichtung handeln, welche eingerichtet ist, um eine Kompressionsenergie in eine kinetische Energie umzuwandeln.

Unabhängig vom jeweiligen tatsächlichen Druck werden im Rahmen der vorliegenden Erfindung die Begriffe Hochdruckdüse und Niederdruckdüse als Bezeichnungen verwendet, welche lediglich zum Ausdruck bringen, dass ein Druck, eine kinetische Energie, ein Impuls oder eine Geschwindigkeit mindestens eines von der Hochdruckdüse erzeugten Strahls des Reinigungsfluids, bei demselben Ausgangsdruck vor einem Passieren der jeweiligen Düse, größer ist als ein Druck bzw. eine kinetische Energie bzw. ein Impuls bzw. eine Geschwindigkeit mindestens eines von der Niederdruckdüse erzeugten Strahls des Reinigungsfluids. Allgemein kann die Hochdruckdüse eingerichtet sein, um das Reinigungsgut mit einem oder mehreren Strahlen des Reinigungsfluids zu aufschlagen, welche einen höheren Druck, eine höhere Geschwindigkeit, einen höheren Impuls oder eine höhere kinetische Energie aufweisen als entsprechende Strahlen, mit denen die Niederdruckdüse das Reinigungsgut bei gleichem Ausgangsdruck vor einem Passieren der jeweiligen Düse beaufschlagt.

Beispielsweise kann die Niederdruckdüse für einen Durchlass eines großen Volumenstroms des Reinigungsfluids eingerichtet sein, wohingegen die Hochdruckdüse für einen Durchlass eines geringeren Volumenstroms eingerichtet sein kann. Beispielsweise kann bei gleichem Ausgangsdruck vor einem Passieren der jeweiligen Düse ein Volumenstrom oder gesamter Volumenstrom des Reinigungsfluids, welches die Niederdruckdüse passiert, um mindestens einen Faktor 1,5, vorzugsweise mindestens einen Faktor 2, insbesondere mindestens einen Faktor 3, mindestens einen Faktor 4 oder sogar mindestens einen Faktor 5, größer sein als ein Volumenstrom oder gesamter Volumenstrom des Reinigungsfluids, welches die Hochdruckdüse passiert.

Beispielsweise kann die Hochdruckdüse einen höheren Strömungswiderstand, insbesondere einen höheren gesamten Strömungswiderstand, aufweisen als die Niederdruckdüse. Als Strömungswiderstand kann beispielsweise das Verhältnis aus einer Druckdifferenz vor und nach der jeweiligen Düse zu einem gesamten Volumenstrom durch die Düse definiert werden. Beispielsweise kann die Hochdruckdüse einen Strömungswiderstand aufweisen, welcher um mindestens einen Faktor 1,5 größer ist als der Strömungswiderstand der Niederdruckdüse, vorzugsweise um mindestens einen Faktor 2 größer oder sogar um mindestens einen Faktor 3, mindestens einen Faktor 4 oder mindestens einen Faktor 5.

Alternativ oder zusätzlich kann die Hochdruckdüse eine im Vergleich zu der Niederdruckdüse kleinere Sprühöffnung aufweisen. Sind mehrere Sprühöffnung und vorgesehen, so kann beispielsweise ein Gesamtquerschnitt der Sprühöffnungen der Hochdruckdüse kleiner sein als ein Gesamtquerschnitt der Sprühöffnungen der Niederdruckdüse. Die Hochdruckdüse kann beispielsweise mindestens einen um einen Faktor 1,5 kleineren Düsenquerschnitt, besonders bevorzugt einen um einen Faktor 2 kleineren Düsenquerschnitt, mindestens einen um einen Faktor 5 kleineren Düsenquerschnitt aufweisen als die Niederdruckdüse.

Unter einem Düsenquerschnitt ist ein Gesamtquerschnitt der mindestens einen Düsenöffnung der Düse zu verstehen, beispielsweise in einer Schnittebene senkrecht zu einer Hauptströmungsrichtung, in welcher das Reinigungsfluid die Düse durchströmt. Sind mehrere Düsenöffnungen in der jeweiligen Düse vorgesehen, so sind deren Düsenquerschnitte jeweils zu addieren.

Weiterhin umfasst die Reinigungsvorrichtung mindestens eine Pumpe zur Förderung des Reinigungsfluids zu dem Düsensystem. Unter einer Pumpe ist allgemein eine Vorrichtung zum Transport und/oder zur Förderung einer Flüssigkeit zu verstehen. Beispielsweise kann eine Antriebsarbeit der Pumpe durch einen Motor, beispielsweise einen Elektromotor, geleistet werden. Über mindestens eine Zuleitung kann der Pumpe das zu fördernde Reinigungsfluid zugeführt werden.

Zur Bereitstellung des Reinigungsfluids kann die Pumpe mit mindestens einem Fluidtank verbunden sein. Der Fluidtank kann mindestens ein Flüssigkeitsreservoir zur Aufnahme des Reinigungsfluids und/oder eines Bestandteils desselben umfassen, beispielsweise kann der Fluidtank als Wassertank ausgebildet sein. Weiterhin kann der Fluidtank mindestens einen Dampferzeuger umfassen. Unter einem Dampferzeuger ist eine Vorrichtung zur Erzeugung von Dampf zu verstehen. Bei Dampf kann es sich beispielsweise um Wasserdampf handeln, beispielsweise von Wasserdampf mit einer Temperatur von mindestens 80 °C, vorzugsweise von mindestens 90 °C und besonders bevorzugt von Wasserdampf von mindestens 100 °C. Auch Dampfgemische sind jedoch grundsätzlich möglich, beispielsweise Dampfgemische aus Wasserdampf mit mindestens einer zusätzlichen Gaskomponente, beispielsweise einem desinfizierenden Gas. Der Dampferzeuger kann ein in dem Fluidtank integriertes oder auch ein separates Bauteil sein.

Die Pumpe ist mehrstufig ausgestaltet. Unter mehrstufig ist zu verstehen, dass die Pumpe mindestens zwei hydraulische Elemente enthält, die bei gleicher Antriebsdrehzahl jeweils eine hydraulische Leistung mit unterschiedlichen hydraulischen Merkmalen bezüglich Druck und Volumenstrom abgeben.

Die Pumpe weist mindestens eine Niederdruckstufe und mindestens eine, der Niederdruckstufe in einer Strömungsrichtung des Reinigungsfluids nachgelagerte, Hochdruckstufe auf. Aus der Niederdruckstufe kann die Pumpe das Reinigungsfluid mit wenig Druck zu dem Düsensystem befördern. In der Hochdruckstufe kann die Pumpe einen höheren Druck erzeugen als in der Niederdruckstufe und beispielsweise gleichzeitig auch nur einen kleineren Volumenstrom des Reinigungsfluids fördern, insbesondere einen um mindestens 50 % kleineren Volumenstrom als in der Niederdruckstufe. Die mindestens eine Niederdruckstufe und die mindestens eine Hochdruckstufe können in einem gemeinsamen Pumpengehäuse angeordnet sein. Das Pumpengehäuse kann aus feststehenden Bauteilen bestehen, die der Führung des zu fördernden Fluids dienen können und die eingerichtet sein können die drehenden Bauteile, wie Antriebswelle und Pumpenlaufräder aufzunehmen. Zusätzlich können im Pumpengehäuse Einbauten vorhanden sein, die die Fluidströmung im Inneren, besonders zwischen den unterschiedlichen Pumpenstufen beeinflussen.

Weiter können die mindestens eine Niederdruckstufe und die mindestens eine Hochdruckstufe durch eine gemeinsame Antriebswelle angetrieben werden. Vorzugsweise kann die gemeinsame Antriebswelle mit einem gemeinsamen Antrieb verbunden sein, beispielsweise einem Elektromotor, dessen Antriebskraft die Antriebswelle auf die Pumpe überträgt. Ein Ausgang der Niederdruckstufe kann mit einem Eingang der Hochdruckstufe verbunden sein.

Weiter sind die Hochdruckdüse und die Niederdruckdüse fluidisch mit der Pumpe verbunden. Beispielsweise kann die Pumpe über eine gemeinsame Zuleitung mit der mindestens einen Hochdruckdüse und der mindestens einen Niederdruckdüse verbunden sein. Von der gemeinsamen Zuleitung können Abzweigungen zu der mindestens einen Hochdruckdüse und der mindestens einen Niederdruckdüse führen.

Bevorzugt kann ein Ausgang der Hochdruckstufe fluidisch mit dem Düsensystem verbunden sein.

Weiterhin ist der Ausgang der Niederdruckstufe über mindestens einen Bypass unter Umgehung der Hochdruckstufe fluidisch mit dem Düsensystem verbunden. Unter dem Bypass kann eine Zuleitung verstanden werden, welche den Ausgang der Niederdruckstufe direkt mit der gemeinsamen Zuleitung zu dem Düsensystem verbindet, so dass das Reinigungsfluid die Hochdruckstufe umgeht. Der Bypass kann mit der mindestens einen Hochdruckstufe und der mindestens einen Niederdruckstufe in einem gemeinsamen Pumpengehäuse angeordnet sein. Der Ausgang der Hochdruckstufe und der Bypass können in einer gemeinsamen Zuleitung zu der Hochdruckdüse und der Niederdruckdüse münden. Die Pumpe kann über eine oder mehrere Hochdruckstufen verfügen. Wenn die Pumpe über mehrere Hochdruckstufen verfügt, so kann der Bypass eingerichtet sein, um eine Hochdruckstufe zu überbrücken oder auch um mehrere Hochdruckstufen oder alle Hochdruckstufen zu überbrücken oder zu umgehen.

In einer Ausführungsform kann die Pumpe mindestens zwei Hochdruckstufen aufweisen, welche in Serie geschaltet sein können. Beispielsweise führt bei zwei in Serie geschalteten Hochdruckstufen der Ausgang einer ersten Hochdruckstufe unmittelbar und ausschließlich in einen Eingang einer zweiten Hochdruckstufe. In dieser Ausführungsform kann der Bypass unter Umgehung der mindestens zwei Hochdruckstufen fluidisch mit dem Düsensystem verbunden sein. Die Reinigungsvorrichtung kann mehrere Hochdruckstufen aufweisen. Bevorzugt kann ein Ausgang einer vorhergehenden Hochdruckstufe direkt zu einem Eingang einer anschließenden Hochdruckstufe führen. Der Ausgang der letzten Hochdruckstufe der Serie kann mit der gemeinsamen Zuleitung zu dem Düsensystem verbunden sein.

In dem Bypass kann mindestens ein Schaltorgan angeordnet sein, welches eingerichtet ist, um eine Strömung des Reinigungsfluids von dem Ausgang der Niederdruckstufe durch den Bypass zu drosseln, und vorzugsweise zu sperren. Unter einem Schaltorgan ist allgemein ein Bauteil zu verstehen, mit dem die Strömung durch dieses Bauteil kontrolliert werden kann, insbesondere ein Ventil.

Das Schaltorgan kann ein passives Schaltorgan umfassen. Unter einem passiven Schaltorgan ist ein Schaltorgan zu verstehen, welches nicht aktiv durch eine Steuerung gesteuert werden kann. Beispielsweise kann das passive Schaltorgan ein druckgesteuertes Schaltorgan sein, insbesondere kann das Schaltorgan ein Rückschlagventil umfassen. Unter einem Rückschlagventil ist ein Bauteil zu verstehen, welches die Strömung des Reinigungsfluids in nur eine Richtung zulässt und in eine andere Richtung absperrt.

Das Schaltorgan kann derart eingerichtet sein, dass ein Durchfluss des Reinigungsfluids durch den Bypass ausschließlich in Richtung zu dem Düsensystem ermöglicht ist. Bevorzugt kann das Rückschlagventil als ein passives Kugel-Rückschlagventil ausgestaltet werden. Unter einem Kugel-Rückschlagventil ist allgemein ein Bauteil zu verstehen, das ein Sperrelement besitzt, das zumindest teilweise kugelförmig ist. Beispielsweise kann das Rückschlagventil, insbesondere das Kugel-Rückschlagventil, mindestens ein Sperrelement aufweisen, welches vorzugsweise zumindest abschnittsweise kugelförmig ausgestaltet ist, wobei das Sperrelement durch mindestens ein Federelement in einen Sitz gepresst wird und dadurch eine Öffnung verschließt, wobei durch einen ausreichenden Gegendruck des Reinigungsfluids das Sperrelement wieder aus seinem Sitz gehoben und die Öffnung freigegeben werden kann. Auch eine andere Ausgestaltung des Schaltorgans ist grundsätzlich möglich. Alternativ oder zusätzlich zu einer vollständigen oder teilweisen Ausgestaltung des Rückschlagventils als Kugel-Rückschlagventil, insbesondere als passives Kugel-Rückschlagventil, sind auch andere Bauformen denkbar. So kann das Rückschlagventil, alternativ oder zusätzlich zu einem Kugel-Rückschlagventil, auch mindestens ein Kegelventil und/oder mindestens ein Klappenventil umfassen. Auch andere Bauformen sind denkbar.

Der Niederdruckdüse ist mindestens ein fluidisches Schaltelement zur Einstellung eines Zuflusses des Reinigungsfluids zu der Niederdruckdüse vorgeschaltet. Das fluidische Schaltelement kann in mindestens einer zu der Niederdruckdüse führenden Abzweigung angeordnet sein. Unter einem fluidischen Schaltelement ist ein Schaltorgan zu verstehen, welches eingerichtet ist, die Niederdruckdüse hydraulisch abzusperren, beispielsweise kann das fluidische Schaltelement mindestens ein Absperrventil umfassen. Das fluidische Schaltelement kann insbesondere ausgewählt sein aus der Gruppe bestehend aus: einem Ventil, einem Hahn und einem Schieber. Die Betätigung des fluidischen Schaltelements kann auf unterschiedliche Arten erfolgen. Beispielsweise kann mindestens ein Antrieb vorgesehen sein, ausgewählt aus der Gruppe bestehend aus: einem elektrischer Antrieb, einem hydraulischen Antrieb, einem pneumatischen Antrieb, einem mechanischen Antrieb, einem elektromagnetischen Antrieb. Besonders bevorzugt kann ein elektromagnetischer Antrieb vorgesehen sein. Auch andere Antriebe sind jedoch denkbar.

Das oben beschriebene Schaltorgan, welches eingerichtet ist, um eine Strömung des Reinigungsfluids von dem Ausgang der Niederdruckstufe durch den Bypass zu drosseln, insbesondere ganz zu verhindern, kann weiter derart eingerichtet sein, dass bei einem geöffneten fluidischen Schaltelement vor der Niederdruckdüse ein Durchfluss durch den Bypass ermöglicht ist und dass bei einem geschlossenen fluidischen Schaltelement ein Durchfluss durch den Bypass verhindert wird. Diese Ausführungsform ist besonders vorteilhaft, da so eine einfache Ansteuerung der Niederdruck- und Hochdruckstufen der Pumpe möglich ist.

Die Reinigungsvorrichtung kann mindestens eine Steuerung aufweisen, die eingerichtet ist, um mindestens ein Reinigungsprogramm durchzuführen. Die Steuerung kann zentral oder dezentral sein. Beispielsweise kann die Steuerung mindestens eine Datenverarbeitungsvorrichtung umfassen, beispielsweise mindestens einen Prozessor, insbesondere einen Mikrocontroller. Weiter kann die Steuerung eine oder mehrere Schnittstellen, beispielsweise mindestens eine Benutzerschnittstelle und/oder mindestens eine elektronische Schnittstelle, aufweisen. Beispielsweise kann die Schnittstelle eine Tastatur und/oder ein Bediendisplay umfassen, über das ein Benutzer ein oder mehrere Parameter, wie beispielsweise eine vorgegebene Reihenfolge von einem Niederdruckbetrieb-Programmschritt und von einem Hochdruckbetrieb-Programmschritt, einstellen kann. Eine elektronische Schnittstelle kann beispielsweise eine Verbindung zu einer anderen Maschine, beispielsweise einem Computer oder Computernetzwerk, bereitstellen, wobei beispielsweise Informationen und/oder Befehle zwischen der Maschine und der Reinigungsvorrichtung oder umgekehrt ausgetauscht werden können. Beispielsweise können auf diese Weise Informationen über eine zu erwartende Verschmutzung an die Reinigungsvorrichtung übermittelt werden. Die Schnittstelle kann unidirektional oder bidirektional sein.

Das Reinigungsprogramm kann mindestens einen Niederdruckbetrieb-Programmschritt und mindestens einen Hochdruckbetrieb-Programmschritt umfassen. In dem mindestens einen Niederdruckbetrieb-Programmschritt des Reinigungsprogramms kann das fluidische Schaltelement geöffnet sein und das Reinigungsgut über die mindestens eine Niederdruckdüse mit dem Reinigungsfluid beaufschlagt werden.

In dem mindestens einen Hochdruckbetrieb-Programmschritt des Reinigungsprogramms kann das fluidische Schaltelement geschlossen sein und das Reinigungsgut ausschließlich über die mindestens eine Hochdruckdüse mit dem Reinigungsfluid beaufschlagt werden. Die Steuerung kann eingerichtet sein, um den Hochdruckbetrieb-Programmschritt mindestens einmal nach dem Niederdruckbetrieb-Programmschritt durchzuführen. Auch eine andere Reihenfolge der Niederdruckbetrieb- und Hochdruckbetrieb-Programmschritte ist grundsätzlich möglich. Auch eine mehrfache Durchführung des Niederdruckbetrieb-Programmschritts und/oder des Hochdruckbetrieb-Programmschritts ist möglich.

Weiter kann die Reinigungsvorrichtung eingerichtet sein, um das Reinigungsgut in mindestens einem Programmschritt mit Dampf zu beaufschlagen. Die Beaufschlagung mit Dampf kann im Anschluss an die Niederdruckbetrieb- und Hochdruckbetrieb-Programmschritte durchgeführt werden. Auch eine andere Reihenfolge der Reinigungsprogrammschritte ist jedoch grundsätzlich möglich.

Weiter weist die Reinigungsvorrichtung mindestens eine Reinigungskammer zur Aufnahme des Reinigungsguts auf. Eine Reinigungskammer im Sinne der vorliegenden Erfindung kann eine vollständig oder teilweise geschlossene Kammer zur Aufnahme des Reinigungsguts sein. Die Reinigungskammer kann insbesondere vollständig oder teilweise durch mindestens ein Gehäuse umgeben sein, beispielsweise ein Gehäuse, welches vollständig oder teilweise aus einem metallischen Material hergestellt ist, beispielsweise einem oder mehreren Blechen aus Edelstahl. Insbesondere kann die Reinigungskammer eine geschlossene Kammer sein, welche vollständig von einem Gehäuse umgegeben ist. Die Reinigungskammer kann insbesondere mindestens eine Öffnung aufweisen, durch welche der zu reinigende Behälter in einen Innenraum der Reinigungskammer eingebracht werden kann. Die Öffnung kann insbesondere verschließbar ausgestaltet sein. Beispielsweise kann die Reinigungskammer mindestens eine Tür zum Öffnen und Schließen der Reinigungskammer und zum Beladen und Entladen der Reinigungskammer mit Reinigungsgut aufweisen. Unter einer Tür ist grundsätzlich eine beliebige Verschlussvorrichtung der Reinigungskammer zu verstehen. Die Tür kann insbesondere ausgewählt sein aus der Gruppe bestehend aus: einer an einer Frontseite der Reinigungsvorrichtung befindlichen Klapptür, insbesondere einer nach unten aufschwenkenden Klapptür; einer auf einer Oberseite der Reinigungsvorrichtung angeordneten Klappe; einer Schiebetür; einer Haube, insbesondere einer nach oben auffahrbaren Haube.

Die Reinigungsvorrichtung kann mindestens eine Halterung zur Aufnahme des Reinigungsguts aufweisen. Unter einer Halterung ist allgemein eine Vorrichtung zu verstehen, welche eingerichtet ist, um den Behälter aufzunehmen und relativ zur Halterung zu fixieren. Beispielsweise kann diese Halterung mindestens eine Schiene umfassen, in welche der Behälter eingeschoben werden kann. Alternativ oder zusätzlich kann die Halterung einen oder mehrere Hohlräume umfassen, in welche der Behälter eingeschoben und/oder eingebracht werden kann. Dabei kann die Reinigungsvorrichtung eine feste Halterung aufweisen, oder auch eine austauschbare Halterung, beispielsweise indem eine Art der Halterung auf den Typ des Behälters angepasst werden kann.

Die Reinigungsvorrichtung kann mindestens einen Abfluss aufweisen. Der Abfluss kann einen Querschnitt, beispielsweise einen Durchmesser oder auch Äquivalentdurchmesser eines Eingangs in den Abfluss, von mindestens 30 mm, vorzugsweise mindestens 50 mm und besonders bevorzugt mindestens 70 mm oder sogar mindestens 100 mm, aufweisen. Die Halterung kann eingerichtet sein, um das Reinigungsgut direkt oder indirekt in den Abfluss zu entleeren. Beispielsweise kann die Halterung eingerichtet sein, um das Reinigungsgut derart zu schwenken, dass dieses in einen Abfluss entleert wird. Das Entleeren kann beispielsweise automatisch erfolgen, beispielsweise vor und/oder während eines Reinigungsprogramms. Beispielsweise kann die Entleerung des Reinigungsguts bei einem Schließen der Tür erfolgen, insbesondere falls die Halterung mit der Tür verbunden ist.

Die Reinigungskammer der Reinigungsvorrichtung kann im Bodenbereich zumindest teilweise trichterförmig und/oder geneigt auf den Abfluss zulaufen.

Insbesondere kann der Abfluss mindestens einen Geruchsverschluss aufweisen. Unter einem Geruchsverschluss ist eine Vorrichtung zu verstehen, mit der Gase aus mindestens einem mit dem Abfluss verbundenen Abflussrohr von dem Inneren der Reinigungskammer ferngehalten werden können. Beispielsweise kann der Geruchsverschluss mindestens einen Siphonbogen aufweisen. Der Siphonbogen kann beispielsweise eine Flüssigkeitsmenge als Geruchsverschluss aufweisen. Zudem kann die Reinigungsvorrichtung vorzugsweise mindestens einen Bypass aufweisen, um Gas und Dampf aus der Reinigungskammer unter Umgehung des Geruchsverschlusses, beispielsweise unter Umgehung des Siphonbogens, in den Abfluss zu leiten, vorzugsweise unter Druck. Dazu kann der Bypass beispielsweise eine oder mehrere Rohrleitungen aufweisen, die die Reinigungskammer mit dem Abfluss unter Umgehung des Geruchsverschlusses verbinden, sowie optional mindestens ein Rückschlagventil und/oder mindestens eine andere Art von Ventil, welches ein Eindringen von Gasen aus dem Abfluss und/oder einem mit dem Abfluss verbundenen Abflussrohr durch den Bypass in die Reinigungskammer ganz oder teilweise verhindert.

In einem weiteren Aspekt wird ein Verfahren zum Reinigen von Reinigungsgut vorgeschlagen. In dem Verfahren wird das Reinigungsgut mittels mindestens eines Düsensystems mit mindestens einem Reinigungsfluid beaufschlagt und eine Pumpe zur Förderung des Reinigungsfluids zu dem Düsensystem verwendet, insbesondere die in einem ersten Aspekt der Erfindung beschriebene mehrstufige Pumpe. Das Düsensystem weist mindestens eine Hochdruckdüse und mindestens eine Niederdruckdüse auf. Bezüglich möglicher Ausgestaltungen und Definitionen der Hochdruckdüse und der Niederdruckdüse kann auf die obige Beschreibung verwiesen werden. Weiter sind die Hochdruckdüse und die Niederdruckdüse fluidisch mit der Pumpe verbunden. Ein Zufluss des Reinigungsfluids zu der Niederdruckdüse wird mittels mindestens eines der Niederdruckdüse vorgeschalteten fluidischen Schaltelements eingestellt.

Das Verfahren kann die Durchführung mindestens eines Reinigungsprogramms umfassen. In mindestens einem Niederdruckbetrieb-Programmschritt des Reinigungsprogramms kann das fluidische Schaltelement geöffnet werden und das Reinigungsgut über die mindestens eine Niederdruckdüse mit dem Reinigungsfluid beaufschlagt werden. In mindestens einem Hochdruckbetrieb-Programmschritt des Reinigungsprogramms kann das fluidische Schaltelement geschlossen werden und das Reinigungsgut über die mindestens eine Hochdruckdüse mit dem Reinigungsfluid beaufschlagt werden, vorzugsweise ausschließlich über die mindestens eine Hochdruckdüse.

Die Pumpe weist mindestens eine Niederdruckstufe und mindestens eine der Niederdruckstufe nachgeschaltete Hochdruckstufe auf. In dem Niederdruckbetrieb-Programmschritt kann Reinigungsfluid aus der Niederdruckstufe mittels mindestens eines Bypasses an der Hochdruckstufe vorbeigeleitet werden und dem Düsensystem zugeführt werden, beispielsweise direkt. In dem Hochdruckbetrieb-Programmschritt kann aufgrund des Schließens des fluidischen Schaltelements ein Druck des Reinigungsfluids an dem Düsensystem im Vergleich zu dem Niederdruckbetrieb-Programmschritt erhöht werden, wodurch das mindestens eine Schaltorgan in dem Bypass geschlossen werden kann und Reinigungsfluid aus der Niederdruckstufe durch die Hochdruckstufe zu dem Düsensystem geleitet werden kann.

In dem vorgeschlagenen Verfahren kann insbesondere die in dem ersten Aspekt der Erfindung vorgeschlagene Reinigungsvorrichtung verwendet werden, beispielsweise nach einem der oben beschriebenen und/oder einer der nachfolgend noch näher beschriebenen Ausführungsformen.

Beispielsweise kann ein Reinigungsprogramm mit einer Reinigung im Niederdruckbetrieb beginnen oder zumindest zu Anfang des Reinigungsprogramms mindestens eine Reinigung im Niederdruckbetrieb umfassen. Dazu kann das fluidische Schaltelement geöffnet sein. Die Pumpe kann von der Steuerung in Betrieb genommen werden, in der Niederdruckstufe laufen und das Reinigungsfluid über die gemeinsame Zuleitung zu der mindestens einen Niederdruckdüse und der mindestens einen Hochdruckdüse fördern. Durch den Einsatz beider Düsen kann sich ein hoher Volumenstrom bei gleichzeitig niedrigem Druck einstellen. In diesem Programmschritt können insbesondere grobe Verschmutzungen oder auch eventuell im Reinigungsgut befindliche Zellstoffe ausgespült werden. Der hohe Volumenstrom ist zudem vorteilhaft, da der an die Reinigungskammer angeschlossene Geruchsverschluss gut durchgespült werden kann. Üblicherweise kann zu dem Durchspülen des Geruchsverschlusses ein Volumenstrom von mindestens 1 l/s erforderlich sein.

In einem anschließenden Reinigungsschritt kann eine Reinigung im Hochdruckbetrieb erfolgen. Dazu wird das fluidische Schaltelement geschlossen. Durch den alleinigen Einsatz der Hochdruckdüsen kann sich ein hoher Druck einstellen. Das Rückschlagventil am Ausgang der Niederdruckstufe kann den Bypass absperren. Die Pumpe kann nun im Hochdruckbetrieb arbeiten, so dass beispielsweise das Reinigungsfluid von der Niederdruckstufe in die Hochdruckstufe geleitet wird, in der sich der Druck weiter erhöhen kann. Das Reinigungsfluid kann unter hohem Druck über die gemeinsame Zuleitung zu der mindestens einen Hochdruckdüse gefördert werden. Das aus der mindestens einen Hochdruckdüse austretende Reinigungsfluid kann, beispielsweise bezogen auf den Volumenstrom, einen hohen Energieinhalt aufweisen, beispielsweise eine hohe kinetische Energie und/oder einen hohen Impuls. In diesem Programmschritt kann eine stark verbesserte Reinigungswirkung, beispielsweise in einer Grenzschicht zwischen Schmutz und Reinigungsgut, erzielt werden gegenüber dem vorhergehenden Programmschritt, vorzugsweise bei gleichzeitig verringertem Volumenstrom.

Das weitere Reinigungsprogramm kann weitere Abfolgen der Niederdruckbetrieb- und Hochdruckbetrieb-Programmschritte aufweisen. Optional können auch Desinfektionsschritte und/oder Trocknungsschritte erfolgen.

Die vorgeschlagene Reinigungsvorrichtung weist insbesondere auch gegenüber dem oben genannten Stand der Technik zahlreiche Unterschiede und Vorteile auf. So werden in DE 198 12 231 A1 unterschiedliche Drücke mit unterschiedlichen Drehzahlen einer Pumpe erreicht. Hierfür ist eine entsprechende Steuerung erforderlich. DE 195 21 21 536 C2 beschreibt einen Hochdruckreiniger mit zwei Pumpen. Dabei ist jedoch die zweite Pumpe als Dosierpumpe für ein Reinigungsmittel ausgestaltet. Trotzdem ist dieses Gerät zur Reinigung mit zwei Drücken im Reinigungsfluid eingerichtet. Der erste, niedrigere Druck wird dabei jedoch nicht durch eine Pumpe im Gerät erzeugt, sondern nur durch den Druck in einer bauseitigen Versorgungsleitung. Dementsprechend sind für den Betrieb des Gerätes bauseitige Maßnahmen erforderlich, welche erfindungsgemäß vermieden werden können. In DE 1 503 853 wird keine zweistufige Kreiselpumpe beschrieben, sondern es wird eine Kreiselpumpe mit einer separaten Kolbenpumpe kombiniert, welche eine separate Düse speist. Die separate Kolbenpumpe besitzt auch einen eigenen separaten Antrieb.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figur 1: eine erfindungsgemäße Reinigungsvorrichtung mit einer Niederdruckdüse und einer Hochdruckdüse; und
- Figur 2: eine erfindungsgemäße Pumpe und ein erfindungsgemäßes Düsensystem.

### Beschreibung der Ausführungsbeispiele

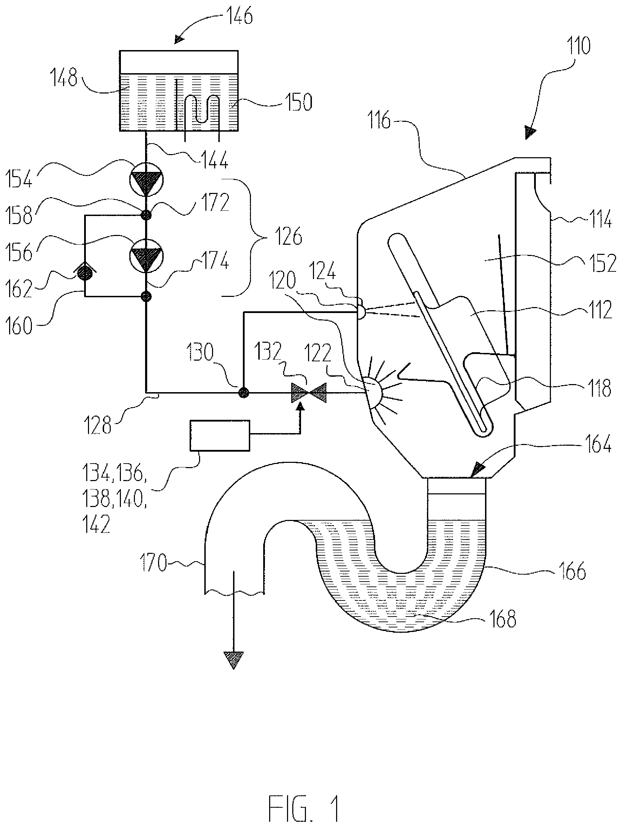

Ein Ausführungsbeispiel einer erfindungsgemäßen Reinigungsvorrichtung 110 zur Reinigung von Reinigungsgut 112 ist in Figur 1 dargestellt. Die Reinigungsvorrichtung 110 kann beispielsweise als eine Reinigungsvorrichtung zum Reinigen von Behältern zur Aufnahme menschlicher Ausscheidungen, insbesondere einem Reinigungs- und Desinfektionsgerät und/oder einem Steckbeckenspüler, ausgestaltet sein. Das Reinigungsgut 112 kann beispielsweise ein Behälter zur Aufnahme menschlicher Ausscheidungen sein. Das Reinigungsgut kann durch eine Tür 114, beispielsweise wie in Figur 1 dargestellt eine Frontklappe, oder eine auf eine andere Weise ausgestaltete Tür, in eine Reinigungskammer 116 eingebracht werden. Das Öffnen und Schließen der Tür 114 kann automatisch und/oder manuell erfolgen. In der Reinigungskammer 116 kann das Reinigungsgut 112 durch eine Halterung 118, beispielsweise ein Drahtgestänge oder eine andere Art von Halterung 118 fixiert werden.

Die Reinigungskammer 116 weist ein Düsensystem 120 auf, um das Reinigungsgut 112 mit einem Reinigungsfluid zu beaufschlagen. Das Reinigungsfluid kann beispielsweise Wasser und/oder eine Reinigungsflüssigkeit mit einem Zusatz von Reinigerkonzentrat und/oder einem chemischen Zusatz sein. Das Düsensystem 120 umfasst mindestens eine Niederdruckdüse 122 und mindestens eine Hochdruckdüse 124. Die Hochdruckdüse 124 weist beispielsweise einen kleineren Düsenquerschnitt und/oder einen höheren Strömungswiderstand auf als die Niederdruckdüse 122. Beispielsweise kann die Hochdruckdüse 124 eine kleinere Sprühöffnung aufweisen als die Niederdruckdüse 122.

Die Reinigungsvorrichtung 110 weist mindestens eine Pumpe 126 auf, welche mit der Niederdruckdüse 122 und der Hochdruckdüse 124 fluidisch verbunden ist. Beispielsweise kann die Pumpe 126 über eine gemeinsame Zuleitung 128 mit der Niederdruckdüse 122 und der Hochdruckdüse 124 verbunden sein. Die gemeinsame Zuleitung 128 kann Abzweigungen 130 zu der Niederdruckdüse 122 und der Hochdruckdüse 124 aufweisen. Die zu der Niederdruckdüse 122 führende Abzweigung weist mindestens ein fluidisches Schaltelement 132, beispielsweise ein Absperrventil und/oder ein Hahn und/oder ein Schieber, auf. Mit dem fluidischen Schaltelement 132 wird der Zufluss des Reinigungsfluids zu der Niederdruckdüse 122 eingestellt.

Die Reinigungsvorrichtung 110 weist eine Steuerung 134 auf, welche eingerichtet ist, das fluidische Schaltelement 132 zu öffnen und zu schließen. Die Steuerung 134 ist in Figur 1 nur schematisch dargestellt. Die Steuerung 134 kann beispielsweise mindestens eine Datenverarbeitung 136 und optional mindestens einen Datenspeicher 138 aufweisen. Zudem kann die Steuerung 134 beispielsweise ein oder mehrere Schnittstellen 140 aufweisen, beispielsweise ein oder mehrere Bedienelemente 142, beispielsweise ausgewählt aus der Gruppe bestehend aus: Schaltern, Tastaturen, Bediendisplays und Anzeigevorrichtungen.

Die Pumpe 126 kann über eine Zuleitung 144 mit einem Fluidtank 146 verbunden sein, welcher mindestens ein Flüssigkeitsreservoir 148 umfasst. Beispielsweise kann die Pumpe 126 mit Reinigungsfluid aus dem Flüssigkeitsreservoir 148 beaufschlagt werden. Optional kann der Fluidtank 146 einen Dampferzeuger 150 aufweisen, um Dampf für einen möglichen Desinfektionsschritt während eines Reinigungsprogramms bereitzustellen.

Weiter kann die Steuerung 134 eingerichtet sein, mindestens ein Reinigungsprogramm durchzuführen. Das Reinigungsprogramm kann mindestens einen Niederdruckbetrieb-Programmschritt und mindestens einen Hochdruckbetrieb-Programmschritt umfassen. Beispielsweise kann ein Hochdruckbetrieb-Programmschritt nach einem Niederdruckbetrieb-Programmschritt erfolgen. Aber auch eine andere Reihenfolge ist möglich. Beispielsweise können mehrere Hochdruckbetrieb-Programmschritte nacheinander durchgeführt werden.

In dem Niederdruckbetrieb-Programmschritt kann das fluidische Schaltelement 132 von der Steuerung 134 geöffnet werden, so dass Reinigungsfluid durch die Niederdruckdüse 122 und die Hochdruckdüse 124 in einen Innenraum 152 der Reinigungskammer 116 eintreten kann. Der Eintritt des Reinigungsfluids in die Reinigungskammer 116 kann, da die Hochdruckdüse 124 und die Niederdruckdüse 122 beide verwendet werden, in einem großen Volumenstrom und unter niedrigem Druck erfolgen. Die Pumpe 126 weist eine Niederdruckstufe 154 und eine Hochdruckstufe 156 auf Die Hochdruckstufe 156 ist hinter der Niederdruckstufe 154 in Strömungsrichtung des Reinigungsfluids angeordnet. Weiter ist ein Ausgang der Niederdruckstufe 158 über einen Bypass 160 unter Umgehung der Hochdruckstufe 156 mit dem Düsensystem 120 verbunden. Der Bypass 160 weist mindestens ein Schaltorgan 162 auf, um den Bypass 160 gegen einen Durchfluss des Reinigungsfluids absperren zu können und einen Durchfluss des Reinigungsfluids nur in Richtung Düsensystem 120 zu erlauben. Bei einem geöffneten fluidischen Schaltelement 132 ist ein Durchfluss durch den Bypass 160 möglich. Der Bypass 160 kann in der gemeinsamen Zuleitung 128 zu dem Düsensystem 120 münden.

Die Reinigungsvorrichtung 110 kann einen Abfluss 164 aufweisen, welcher beispielsweise am Boden der Reinigungskammer 116 angeordnet sein kann. Die Halterung 118 kann eingerichtet sein, das Reinigungsgut 112 in den Abfluss 164 zu entleeren. Vorzugsweise kann der Abfluss 164 einen Geruchsverschluss 166 aufweisen, beispielsweise einen Siphonbogen. Der Geruchsverschluss 166 kann beispielsweise einen Flüssigkeitsvorrat 168 als Geruchsverschluss aufweisen. Der Geruchsverschluss 166 kann beispielsweise mit mindestens einem Abflussrohr 170 verbunden sein. Der große Volumenstrom des Reinigungsfluids in dem Niederdruckbetrieb-Programmschritt kann für eine gute Durchspülung des Geruchsverschlusses 166 sorgen.

In dem Hochdruckbetrieb-Programmschritt kann das fluidische Schaltelement 132 von der Steuerung 134 geschlossen werden, so dass das Reinigungsfluid nur durch die Hochdruckdüse 124 in den Innenraum 152 der Reinigungskammer 116 eintreten kann. Mit der Erhöhung des Systemdrucks schließt sich das Schaltorgan 162 im Bypass 160, so dass kein Reinigungsfluid vom Ausgang der Hochdruckstufe zum Eingang der Hochdruckstufe strömt. Der Ausgang der Niederdruckstufe 158 ist mit einem Eingang der Hochdruckstufe 172 verbunden. Ein Ausgang der Hochdruckstufe 174 kann in der gemeinsamen Zuleitung 128 zu dem Düsensystem 120 münden. Das Reinigungsfluid tritt während des Hochdruckbetrieb-Programmschritts unter einem kleinen Volumenstrom und hohen Druck aus der Hochdruckdüse 124 in den Innenraum 152 der Reinigungskammer 116 ein. Dadurch kann eine verbesserte Reinigungsleistung im Vergleich zu einer Reinigungsleistung in dem Niederdruckbetrieb-Programmschritt erreicht werden.

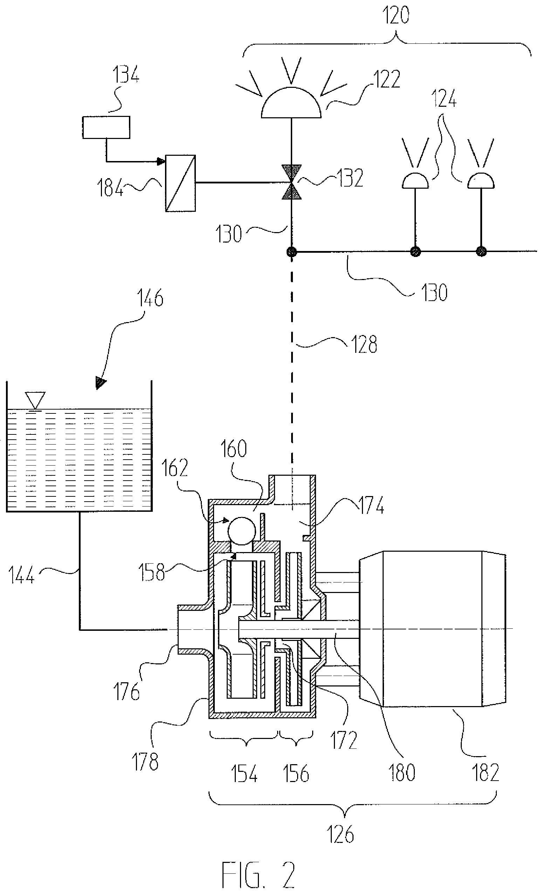

Figur 2 zeigt ein Ausführungsbeispiel einer erfindungsgemäßen Pumpe 126. Ein Eingang 176 der Pumpe 126 kann über die Zuleitung 144 mit dem Fluidtank 146 verbunden sein.

Die Niederdruckstufe 154 und die Hochdruckstufe 156 können in einem gemeinsamen Pumpengehäuse 178 angeordnet sein. Die Niederdruckstufe 154 kann ein großes Volumen und große Strömungsquerschnitte in der Pumpe 126 aufweisen, wohingegen die Hochdruckstufe 156 ein relativ kleineres Volumen und relativ kleinere Strömungsquerschnitte in der Pumpe 126 aufweisen kann. Die Niederdruckstufe 154 und die Hochdruckstufe 156 können durch eine gemeinsame Antriebswelle 180 angetrieben werden. Die Antriebswelle 180 kann durch einen Antrieb, beispielsweise einen Motor 182, insbesondere einen Elektromotor, angetrieben werden. Eine solche Ausgestaltung von einem Niederdrucksystem und einem Hochdrucksystem in einer Baueinheit ist besonders bevorzugt, da ein verringerter Bauaufwand, eine verringerte Anzahl an Bauteilen und ein geringerer Platzbedarf im Vergleich zu Niederdrucksystemen und Hochdrucksystemen aus dem Stand der Technik benötigt wird.

Das Reinigungsfluid kann von dem Fluidtank 146 über die Zuleitung 144 zu dem Eingang der Pumpe 176 transportiert werden. Für einen Niederdruckbetrieb-Programmschritt übergibt die Steuerung 134 beispielsweise einen Programmparameter, beispielsweise einen Programmparameter, welcher den Niederdruckbetrieb-Programmschritt kennzeichnet, an ein Steuerelement 184 des fluidischen Schaltelements 132. Das Steuerelement 184 öffnet das fluidische Schaltelement 132. Das Reinigungsfluid kann von dem Eingang der Pumpe 176 in die Niederdruckstufe 154 gelangen. Am Ausgang der Niederdruckstufe 154 kann der Bypass 160 mit dem Schaltorgan 162 abzweigen. Das Schaltorgan 162 des Bypasses 160 kann den direkten Ausgang der Niederdruckstufe zum Düsensystem 120 hin sperren. Das Schaltorgan 162 kann beispielsweise als ein passives Schaltorgan, vorzugsweise als ein druckgesteuertes Schaltorgan, besonders bevorzugt als ein Rückschlagventil, insbesondere als ein Kugel-Rückschlagventil, ausgestaltet sein. Bei geöffnetem fluidischen Schaltelement 132 kann das Reinigungsfluid die Niederdruckstufe 154 durch den Bypass 160 verlassen und durch die gemeinsame Zuleitung 128 zu der Niederdruckdüse 122 und den Hochdruckdüsen 124 gelangen.

Für einen Hochdruckbetrieb-Programmschritt übergibt die Steuerung 134 einen Programmparameter, beispielsweise einen Programmparameter, welcher den Hochdruckbetrieb-Programmschritt kennzeichnet, an das Steuerelement 184 des fluidischen Schaltelements 132. Das Steuerelement 184 schließt das fluidische Schaltelement 132. Bei geschlossenem fluidischen Schaltelement 132 kann sich der Druck im Düsensystem 120 erhöhen, und das Schaltorgan 162 kann den Ausgang der Niederdruckstufe 158 absperren. Das Reinigungsfluid kann die Pumpe 126 nicht über den Bypass 160 verlassen, sondern gelangt über den Eingang der Hochdruckstufe 172, welcher mit dem Ausgang der Niederdruckstufe 154 verbunden ist, in die Hochdruckstufe 156. Das Reinigungsfluid kann von dem Ausgang der Hochdruckstufe 174 über die gemeinsame Zuleitung 128 zu den Hochdruckdüsen 124 gelangen. Diese Ausgestaltungsform ist besonders bevorzugt, da eine einfache Steuerung des Niederdrucksystems und des Hochdrucksystems möglich ist.

Weitere Ausführungsformen, beispielsweise Ausführungsformen, in denen die Pumpe 126 mehr als eine Hochdruckstufe 156 aufweist, sind möglich. Die mindestens zwei Hochdruckstufen 156 können beispielsweise in Serie angeordnet werden. Diese Anordnung in Serie kann zu einer weiteren Druckerhöhung führen. Der Ausgang einer vorherigen Hochdruckstufe kann direkt in einen Eingang der folgenden Hochdruckstufe führen. Der Ausgang der letzten Hochdruckstufe 174 der Serie kann mit der gemeinsamen Zuleitung 128 verbunden sein. Der Bypass 160 kann, in der Ausführungsform, in der die Pumpe 126 mehr als eine Hochdruckstufe 156 aufweist, alle Hochdruckstufen 156 umgehen.

### Bezugszeichenliste

- 110: Reinigungsvorrichtung
- 112: Reinigungsgut
- 114: Tür
- 116: Reinigungskammer
- 118: Halterung
- 120: Düsensystem
- 122: Niederdruckdüse
- 124: Hochdruckdüse
- 126: Pumpe
- 128: gemeinsame Zuleitung
- 130: Abzweigungen
- 132: fluidisches Schaltelement
- 134: Steuerung
- 136: Datenverarbeitung
- 138: Datenspeicher
- 140: Schnittstellen
- 142: Bedienelemente
- 144: Zuleitung
- 146: Fluidtank
- 148: Flüssigkeitsreservoir
- 150: Dampferzeuger
- 152: Innenraum
- 154: Niederdruckstufe
- 156: Hochdruckstufe
- 158: Ausgang der Niederdruckstufe
- 160: Bypass
- 162: Schaltorgan
- 164: Abfluss
- 166: Geruchsverschluss
- 168: Flüssigkeitsvorrat
- 170: Abflussrohr
- 172: Eingang der Hochdruckstufe
- 174: Ausgang der Hochdruckstufe
- 176: Eingang der Pumpe

- 178: Pumpengehäuse
- 180: gemeinsame Antriebswelle
- 182: Motor
- 184: Steuerelement

## Patentansprüche

1. Reinigungsvorrichtung (110) zum Reinigen von Reinigungsgut (112), wobei die Reinigungsvorrichtung (110) ausgewählt ist aus der Gruppe bestehend aus einer Geschirrspülmaschine und einem Reinigungs- und Desinfektionsgerät, wobei die Reinigungsvorrichtung (110) mindestens ein Düsensystem (120) zur Beaufschlagung des Reinigungsguts (112) mit mindestens einem Reinigungsfluid umfasst, wobei die Reinigungsvorrichtung (110) weiterhin mindestens eine Pumpe (126) zur Förderung des Reinigungsfluids zu dem Düsensystem (120) umfasst, wobei das Düsensystem (120) mindestens eine Hochdruckdüse (124) und mindestens eine Niederdruckdüse (122) aufweist, wobei die Hochdruckdüse (124) und die Niederdruckdüse (122) fluidisch mit der Pumpe (126) verbunden sind, wobei der Niederdruckdüse (122) mindestens ein fluidisches Schaltelement (132) zur Einstellung eines Zuflusses des Reinigungsfluids zu der Niederdruckdüse (122) vorgeschaltet ist, wobei die Pumpe (126) mehrstufig ausgestaltet ist, wobei die Pumpe (126) mindestens eine Niederdruckstufe (154) und mindestens eine der Niederdruckstufe (154) in einer Strömungsrichtung des Reinigungsfluids nachgelagerte Hochdruckstufe (156) aufweist, wobei ein Ausgang der Niederdruckstufe (158) mit einem Eingang der Hochdruckstufe (172) verbunden ist, wobei ein Ausgang der Hochdruckstufe (174) fluidisch mit dem Düsensystem (120) verbunden ist, wobei der Ausgang der Niederdruckstufe (158) weiterhin über mindestens einen Bypass (160) unter Umgehung der Hochdruckstufe (156) fluidisch mit dem Düsensystem (120) verbunden ist.

2. Reinigungsvorrichtung (110) nach dem vorhergehenden Anspruch, wobei die Pumpe (126) über eine gemeinsame Zuleitung (128) mit der mindestens einen Hochdruckdüse (124) und der mindestens eine Niederdruckdüse (122) verbunden ist.

3. Reinigungsvorrichtung (110) nach dem vorhergehenden Anspruch, wobei von der gemeinsamen Zuleitung (128) Abzweigungen (130) zu der mindestens einen Hochdruckdüse (124) und der mindestens eine Niederdruckdüse (122) führen.

4. Reinigungsvorrichtung (110) nach dem vorhergehenden Anspruch, wobei das fluidische Schaltelement (132) in mindestens einer zu der Niederdruckdüse (122) führenden Abzweigung (130) angeordnet ist.

5. Reinigungsvorrichtung (110) nach einem der vorhergehenden Ansprüche, wobei die mindestens eine Niederdruckstufe (154) und die mindestens eine Hochdruckstufe (156) durch eine gemeinsame Antriebswelle (180) angetrieben werden.

6. Reinigungsvorrichtung (110) nach einem der vorhergehenden Ansprüche, wobei der Ausgang der Hochdruckstufe (174) und der Bypass (160) in einer gemeinsamen Zuleitung (128) zu der Hochdruckdüse (124) und der Niederdruckdüse (122) münden.

7. Reinigungsvorrichtung (110) nach einem der vorhergehenden Ansprüche, wobei der Bypass (160) mit der mindestens einen Hochdruckstufe (156) und der mindestens einen Niederdruckstufe (154) in einem gemeinsamen Pumpengehäuse (178) angeordnet ist.

8. Reinigungsvorrichtung (110) nach einem der vorhergehenden Ansprüche, wobei in dem Bypass (160) mindestens ein Schaltorgan (162) angeordnet ist.

9. Reinigungsvorrichtung (110) nach dem vorhergehenden Anspruch, wobei das Schaltorgan (162) derart eingerichtet ist, dass bei geöffnetem fluidischem Schaltelement (132) ein Durchfluss durch den Bypass (160) ermöglicht ist und dass bei geschlossenem fluidischem Schaltelement (132) ein Durchfluss durch den Bypass (160) verhindert wird.

10. Reinigungsvorrichtung (110) nach einem der vorhergehenden Ansprüche, wobei die Reinigungsvorrichtung (110) mindestens eine Steuerung (134) aufweist, wobei die Steuerung (134) eingerichtet ist, um mindestens ein Reinigungsprogramm durchzuführen, wobei in mindestens einem Niederdruckbetrieb-Programmschritt des Reinigungsprogramms das fluidische Schaltelement (132) geöffnet ist und das Reinigungsgut (112) über die mindestens eine Niederdruckdüse (122) mit dem Reinigungsfluid beaufschlagbar ist, wobei in mindestens einem Hochdruckbetrieb-Programmschritt des Reinigungsprogramms das fluidische Schaltelement (132) geschlossen ist und das Reinigungsgut (112) ausschließlich über die mindestens eine Hochdruckdüse (124) mit dem Reinigungsfluid beaufschlagbar ist.

11. Verfahren zur Reinigung von Reinigungsgut (112), wobei das Reinigungsgut (112) mittels mindestens eines Düsensystems (120) mit mindestens einem Reinigungsfluid beaufschlagt wird, wobei eine Pumpe (126) zur Förderung des Reinigungsfluids zu dem Düsensystem (120) verwendet wird, wobei das Düsensystem (120) mindestens eine Hochdruckdüse (124) und mindestens eine Niederdruckdüse (122) aufweist, wobei die Hochdruckdüse (124) und die Niederdruckdüse (122) fluidisch mit der Pumpe (126) verbunden sind, wobei ein Zufluss des Reinigungsfluids zu der Niederdruckdüse (122) mittels mindestens eines der Niederdruckdüse (122) vorgeschalteten fluidischen Schaltelements (132) eingestellt wird, wobei die Pumpe (126) mehrstufig ausgestaltet ist, wobei die Pumpe (126) mindestens eine Niederdruckstufe (154) und mindestens eine der Niederdruckstufe (154) in einer Strömungsrichtung des Reinigungsfluids nach gelagerte Hochdruckstufe (156) aufweist, wobei ein Ausgang der Niederdruckstufe (158) mit einem Eingang der Hochdruckstufe (172) verbunden ist, wobei ein Ausgang der Hochdruckstufe (174) fluidisch mit dem Düsensystem (120) verbunden ist, wobei der Ausgang der Niederdruckstufe (158) weiterhin über mindestens einen Bypass (160) unter Umgehung der Hochdruckstufe (156) fluidisch mit dem Düsensystem (120) verbunden ist.

12. Verfahren nach dem vorhergehenden Anspruch, wobei das Verfahren die Durchführung mindestens eines Reinigungsprogramms umfasst, wobei in mindestens einem Niederdruckbetrieb-Programmschritt des Reinigungsprogramms das fluidische Schaltelement (132) geöffnet ist und das Reinigungsgut (112) über die mindestens eine Niederdruckdüse (122) mit dem Reinigungsfluid beaufschlagt wird, wobei in mindestens einem Hochdruckbetrieb-Programmschritt des Reinigungsprogramms das fluidische Schaltelement (132) geschlossen ist und das Reinigungsgut (112) ausschließlich über die mindestens eine Hochdruckdüse (124) mit dem Reinigungsfluid beaufschlagt wird.

13. Verfahren nach dem vorhergehenden Anspruch, wobei die Pumpe (126) die mindestens eine Niederdruckstufe (154) und die mindestens eine der Niederdruckstufe (154) nachgeschaltete Hochdruckstufe (156) aufweist, wobei in dem Niederdruckbetrieb-Programmschritt Reinigungsfluid aus der Niederdruckstufe (154) mittels des mindestens eines Bypasses (160) an der Hochdruckstufe (156) vorbei geleitet wird und dem Düsensystem (120) zugeführt wird, wobei in dem Hochdruckbetrieb-Programmschritt aufgrund des Schließens des fluidischen Schaltelements (132) ein Druck des Reinigungsfluids an dem Düsensystem (120) im Vergleich zu dem Niederdruckbetrieb-Programmschritt erhöht wird, wodurch mindestens ein Schaltorgan (162) in dem Bypass (160) geschlossen wird und Reinigungsfluid aus der Niederdruckstufe (154) durch die Hochdruckstufe (156) zu dem Düsensystem (120) geleitet wird.

## Claims

1. Cleaning device (110) for cleaning items to be cleaned (112), wherein the cleaning device (110) is selected from the group consisting of a dishwasher and cleaning and disinfecting equipment, wherein the cleaning device (110) includes at least one nozzle system (120) for acting upon the items to be cleaned (112) with at least one cleaning fluid, wherein the cleaning device (110) additionally includes at least one pump (126) for conveying the cleaning fluid to the nozzle system (120), wherein the nozzle system (120) comprises at least one high pressure nozzle (124) and at least one low pressure nozzle (122), wherein the high pressure nozzle (124) and the low pressure nozzle (122) are connected fluidically to the pump (126), wherein at least one fluidic switching element (132) for adjusting an inflow of the cleaning fluid to the low pressure nozzle (122) is connected upstream of the low pressure nozzle (122), wherein the pump (126) is developed in multiple stages, wherein the pump (126) comprises at least one low pressure stage (154) and at least one high pressure stage (156) which is mounted downstream of the low pressure stage (154) in a direction of flow of the cleaning fluid, wherein an outlet of the low pressure stage (158) is connected to an inlet of the high pressure stage (172), wherein an outlet of the high pressure stage (174) is connected fluidically to the nozzle system (120), wherein the outlet of the low pressure stage (158) is additionally connected fluidically to the nozzle system (120) by means of a bypass (160) by bypassing the high pressure stage (156).

2. Cleaning device (110) according to the preceding claim, wherein the pump (126) is connected to the at least one high pressure nozzle (124) and the at least one low pressure nozzle (122) via a common supply line (128).

3. Cleaning device (110) according to the preceding claim, wherein branches (130) lead to the at least one high pressure nozzle (124) and to the at least one low pressure nozzle (122) from the common supply line (128).

4. Cleaning device (110) according to the preceding claim, wherein the fluidic switching element (132) is arranged in at least one branch (130) which leads to the low pressure nozzle (122).

5. Cleaning device (110) according to one of the preceding claims, wherein the at least one low pressure stage (154) and the at least one high pressure stage (156) are driven by a common drive shaft (180).

6. Cleaning device (110) according to one of the preceding claims, wherein the outlet of the high pressure stage (174) and the bypass (160) open out in a common feed line (128) to the high pressure nozzle (124) and to the low pressure nozzle (122).

7. Cleaning device (110) according to one of the preceding claims, wherein the bypass (160) is arranged with the at least one high pressure stage (156) and the at least one low pressure stage (154) in a common pump housing (178).

8. Cleaning device (110) according to one of the preceding claims, wherein at least one switching member (162) is arranged in the bypass (160).

9. Cleaning device (110) according to the preceding claim, wherein the switching member (162) is configured in such a manner that when the fluidic switching element (132) is open, flow through the bypass (160) is made possible and in that when the fluidic switching element (132) is closed flow through the bypass (160) is prevented.

10. Cleaning device (110) according to one of the preceding claims, wherein the cleaning device (110) comprises at least one control means (134), wherein the control means (134) is configured to carry out at least one cleaning program, wherein in at least one low pressure mode program step of the cleaning program the fluidic switching element (132) is open and the items to be cleaned (112) can be acted upon with the cleaning fluid by means of the at least one low pressure nozzle (122), wherein in at least one high pressure mode program step of the cleaning program the fluidic switching element (132) is closed and the items to be cleaned (112) can be acted upon with the cleaning fluid exclusively by means of the at least one high pressure nozzle (124).

11. Method for cleaning items to be cleaned (112), wherein the items to be cleaned (112) are acted upon with at least one cleaning fluid by means of at least one nozzle system (120), wherein a pump (126) is used to convey the cleaning fluid to the nozzle system (120), wherein the nozzle system (120) comprises at least one high pressure nozzle (124) and at least one low pressure nozzle (122), wherein the high pressure nozzle (124) and the low pressure nozzle (122) are connected fluidically to the pump (126), wherein an inflow of the cleaning fluid to the low pressure nozzle (122) is adjusted by means of at least one fluidic switching element (132) which is connected upstream of the low pressure nozzle (122), wherein the pump (126) is developed with multiple stages, wherein the pump (126) comprises at least one low pressure stage (154) and at least one high pressure stage (156) which is mounted downstream of the low pressure stage (154) in a direction of flow of the cleaning fluid, wherein an outlet of the low pressure stage (158) is connected to an inlet of the high pressure stage (172), wherein an outlet of the high pressure stage (174) is connected fluidically to the nozzle system (120), wherein the outlet of the low pressure stage (158) is additionally connected fluidically to the nozzle system (120) by means of at least one bypass (160) by bypassing the high pressure stage (156).

12. Method according to the preceding claim, wherein the method includes the carrying out of at least one cleaning program, wherein in at least one low pressure mode program step of the cleaning program the fluidic switching element (132) is open and the items to be cleaned (112) are acted upon with the cleaning fluid by means of the at least one low pressure nozzle (122), wherein in at least one high pressure mode program step of the cleaning program the fluidic switching element (132) is closed and the items to be cleaned (112) are acted upon with the cleaning fluid exclusively by means of the at least one high pressure nozzle (124).

13. Method according to the preceding claim, wherein the pump (126) comprises the at least one low pressure stage (154) and the at least one high pressure stage (156) which is connected downstream of the low pressure stage (154), wherein in the low pressure mode program step cleaning fluid is conducted from the low pressure stage (154) past the high pressure stage (156) by means of the at least one bypass (160) and is supplied to the nozzle system (120), wherein in the high pressure mode program step, on account of the closing of the fluidic switching element (132), a pressure of the cleaning fluid in the nozzle system (120) is increased compared to the low pressure mode program step, as a result of which at least one switching member (162) in the bypass (160) is closed and cleaning fluid is conducted from the low pressure stage (154) through the high pressure stage (156) to the nozzle system (120).

## Revendications

1. Dispositif de nettoyage (110) pour nettoyer un produit à nettoyer (112), le dispositif de nettoyage (110) étant choisi parmi le groupe constitué d'un lave-vaisselle et d'un appareil de nettoyage et désinfection, le dispositif de nettoyage (110) comprenant au moins un système de buses (120) pour solliciter le produit à nettoyer (112) avec au moins un fluide de nettoyage, le dispositif de nettoyage (110) comprenant en outre au moins une pompe (126) pour transporter le fluide de nettoyage vers le système de buses (120), le système de buses (120) présentant au moins une buse haute pression (124) et au moins une buse basse pression (122), la buse haute pression (124) et la buse basse pression (122) étant reliées fluidiquement à la pompe (126), au moins un élément de commutation fluidique (132) étant monté en amont de la buse basse pression (122) pour ajuster un afflux de fluide de nettoyage à la buse basse pression (122), la pompe (126) étant réalisée avec plusieurs étages, la pompe (126) présentant au moins un étage basse pression (154) et au moins un étage haute pression (156) monté en aval de l'étage basse pression (154) dans un sens d'écoulement du fluide de nettoyage, une sortie de l'étage basse pression (158) étant reliée à une entrée de l'étage haute pression (172), une sortie de l'étage haute pression (174) étant reliée fluidiquement au système de buses (120), la sortie de l'étage basse pression (158) étant en outre reliée fluidiquement au système de buses (120) par le biais d'au moins une dérivation (160) en contournant l'étage haute pression (156).

2. Dispositif de nettoyage (110) selon la revendication précédente, dans lequel la pompe (126) est reliée par le biais d'une conduite d'amenée commune (128) à l'au moins une buse haute pression (124) et à l'au moins une buse basse pression (122).

3. Dispositif de nettoyage (110) selon la revendication précédente, dans lequel des branchements (130) conduisent de la conduite d'amenée commune (128) à l'au moins une buse haute pression (124) et à l'au moins une buse basse pression (122).

4. Dispositif de nettoyage (110) selon la revendication précédente, dans lequel l'élément de commutation fluidique (132) est disposé dans au moins un branchement (130) conduisant à la buse basse pression (122).

5. Dispositif de nettoyage (110) selon l'une quelconque des revendications précédentes, dans lequel l'au moins un étage basse pression (154) et l'au moins un étage haute pression (156) sont entraînés par un arbre d'entraînement commun (180).

6. Dispositif de nettoyage (110) selon l'une quelconque des revendications précédentes, dans lequel la sortie de l'étage haute pression (174) et la dérivation (160) débouchent dans une conduite d'amenée commune (128) allant à la buse haute pression (124) et à la buse basse pression (122).

7. Dispositif de nettoyage (110) selon l'une quelconque des revendications précédentes, dans lequel la dérivation (160) est disposée avec l'au moins un étage haute pression (156) et l'au moins un étage basse pression (154) dans un boîtier de pompe commun (178).

8. Dispositif de nettoyage (110) selon l'une quelconque des revendications précédentes, dans lequel au moins un organe de commutation (162) est disposé dans la dérivation (160).

9. Dispositif de nettoyage (110) selon la revendication précédente, dans lequel l'organe de commutation (162) est prévu de telle sorte que lorsque l'élément de commutation fluidique (132) est ouvert, un passage à travers la dérivation (160) est permis et que lorsque l'élément de commutation fluidique (132) est fermé, un passage à travers la dérivation (160) est empêché.

10. Dispositif de nettoyage (110) selon l'une quelconque des revendications précédentes, dans lequel le dispositif de nettoyage (110) présente au moins une commande (134), la commande (134) étant prévue pour effectuer au moins un programme de nettoyage, l'élément de commutation fluidique (132) étant ouvert dans au moins une étape de programme de fonctionnement basse pression du programme de nettoyage et le produit à nettoyer (112) pouvant être sollicité avec le fluide de nettoyage par le biais de l'au moins une buse basse pression (122), dans au moins une étape de programme de fonctionnement haute pression du programme de nettoyage, l'élément de commutation fluidique (132) étant fermé et le produit à nettoyer (112) pouvant être sollicité exclusivement par le biais de l'au moins une buse haute pression (124) avec le fluide de nettoyage.

11. Procédé pour le nettoyage de produit à nettoyer (112), le produit à nettoyer (112) étant sollicité avec au moins un fluide de nettoyage au moyen d'au moins un système de buses (120), une pompe (126) étant utilisée pour tansporter le fluide de nettoyage vers le système de buses (120), le système de buses (120) présentant au moins une buse haute pression (124) et au moins une buse basse pression (122), la buse haute pression (124) et la buse basse pression (122) étant reliées fluidiquement à la pompe (126), un afflux du fluide de nettoyage vers la buse basse pression (122) étant ajusté au moyen d'au moins un élément de commutation fluidique (132) monté en amont de la buse basse pression (122), la pompe (126) étant réalisée avec plusieurs étages, la pompe (126) présentant au moins un étage basse pression (154) et au moins un étage haute pression (156) monté en aval de l'étage basse pression (154) dans un sens d'écoulement du fluide de nettoyage, une sortie de l'étage basse pression (158) étant reliée à une entrée de l'étage haute pression (172), une sortie de l'étage haute pression (174) étant reliée fluidiquement au système de buses (120), la sortie de l'étage basse pression (158) étant en outre reliée fluidiquement au système de buses (120) par le biais d'au moins une dérivation (160) en contournant l'étage haute pression (156).

12. Procédé selon la revendication précédente, dans lequel le procédé comprend la mise en oeuvre d'au moins un programme de nettoyage, l'élément de commutation fluidique (132) étant ouvert dans au moins une étape de programme de fonctionnement basse pression du programme de nettoyage et le produit à nettoyer (112) étant sollicité avec le fluide de nettoyage par le biais de l'au moins une buse basse pression (122), dans au moins une étape de programme de fonctionnement haute pression du programme de nettoyage, l'élément de commutation fluidique (132) étant fermé et le produit à nettoyer (112) étant sollicité exclusivement par le biais de l'au moins une buse haute pression (124) avec le fluide de nettoyage.

13. Procédé selon la revendication précédente, dans lequel la pompe (126) présente l'au moins un étage basse pression (154) et l'au moins un étage haute pression (156) monté en aval de l'étage basse pression (154), dans lequel, dans l'étape de programme de fonctionnement basse pression, du fluide de nettoyage est guidé hors de l'étage basse pression (154) au moyen de l'au moins une dérivation (160) devant l'étage haute pression (156) et est acheminé au système de buses (120), dans l'étape de programme de fonctionnement haute pression, du fait de la fermeture de l'élément de commutation fluidique (132), une pression du fluide de nettoyage au niveau du système de buses (120) est augmentée par rapport à l'étape de programme de fonctionnement basse pression, de sorte qu'au moins un organe de commutation (162) dans la dérivation (160) soit fermé et que du fluide de nettoyage soit guidé hors de l'étage basse pression (154) à travers l'étage haute pression (156) jusqu'au système de buses (120).
